(19) **Europäisches Patentamt**
**European Patent Office**
**Office européen des brevets**

(11) **EP 4 310 256 B1**

(12) **EUROPEAN PATENT SPECIFICATION**

(45) Date of publication and mention
of the grant of the patent:
**04.02.2026 Bulletin 2026/06**

(21) Application number: **22815585.9**

(22) Date of filing: **24.02.2022**

(51) International Patent Classification (IPC):
**E02D 1/00** *(2006.01)* **E02F 9/20** *(2006.01)*
**E02F 9/26** *(2006.01)*

(52) Cooperative Patent Classification (CPC):
**E02F 9/264; E02F 3/307; E02F 9/261;** E02F 3/435;
E02F 9/205

(86) International application number:
**PCT/JP2022/007691**

(87) International publication number:
**WO 2022/254826 (08.12.2022 Gazette 2022/49)**

(54) **SOIL QUALITY INFORMATION ACQUISITION SYSTEM AND WORK MACHINE PROVIDED WITH SAME**

SYSTEM ZUR ERFASSUNG VON BODENQUALITÄTSINFORMATIONEN UND DAMIT AUSGESTATTETE ARBEITSMASCHINE

SYSTÈME D'ACQUISITION D'INFORMATIONS DE QUALITÉ DE SOL ET ENGIN DE CHANTIER ÉQUIPÉ DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.06.2021 JP 2021093728**

(43) Date of publication of application:
**24.01.2024 Bulletin 2024/04**

(73) Proprietor: **Kobelco Construction Machinery Co., Ltd.**
**Hiroshima-shi, Hiroshima 731-5161 (JP)**

(72) Inventors:
• **OKIMOTO, Sho**
**Hiroshima-shi, Hiroshima 731-5161 (JP)**

• **SHIMAZU, Yasuhiko**
**Hiroshima-shi, Hiroshima 731-5161 (JP)**
• **YAMASHITA, Koji**
**Hiroshima-shi, Hiroshima 731-5161 (JP)**

(74) Representative: **TBK**
**Bavariaring 4-6**
**80336 München (DE)**

(56) References cited:
WO-A1-2019/009341 JP-A- 2018 021 885
JP-A- 2018 111 950 JP-A- 2019 127 682
JP-A- 2019 163 621 JP-A- 2019 163 621
JP-A- 2020 037 837 JP-A- 2020 153 731
JP-A- H09 184 128 US-A1- 2006 265 914

## Description

### Technical Field

The present invention relates to a soil quality information acquisition system and a working machine including the system.

### Background Art

A working machine which excavates the ground on a worksite has been conventionally known. The working machine includes a lower traveling body travelable on the ground, an upper body mounted on the lower traveling body, and a working attachment supported by the upper body. In a case where the working machine is in the form of a hydraulic excavator, the working attachment has a bucket at a distal end thereof. The working machine can excavate the ground with the bucket being in contact with the ground.

Patent Literature 1 discloses an excavator including: a sensor attached to a working attachment; and a hardness estimation part that estimates the hardness of the ground on the basis of a detection value from the sensor. The hardness estimation part estimates the hardness of the ground on the basis of data stored in advance and a detection value from the sensor obtained in a predetermined operation performed by a distal end of the working attachment on contact with the ground at a predetermined speed and at a predetermined angle.

Patent Literature 2 discloses a measurement device including: a multi-axial motion sensor mounted on a construction device; a measurement part that measures hardness information about a construction target on the basis of a counterforce accompanied by an impact detected by the multi-axial motion sensor at construction; a position information acquisition part that acquires position information about the construction device; and a communication part that transmits the hardness information and the position information to an outside.

### Citation List

### Patent Literature

Patent Literature 1: Japanese Unexamined Patent Publication No. 2019-163621
Patent Literature 2: Japanese Unexamined Patent Publication No. 2018-111950

Each of the technologies described in Patent Literatures 1 and 2 includes periodically detecting peripheral soil quality information at predetermined time intervals in a work, and thus has a drawback that a storage part needs a large capacity to store the soil quality information.

## Summary of Invention

An object of the present invention is to provide a soil quality information acquisition system that enables acquisition of soil quality information for a worksite at an appropriate time, and a working machine including the system.

The present invention provides a soil quality information acquisition system for use in a working machine including: a machine body having a traveling part travelable on a ground; and a working attachment attached to the machine body movably relative to the machine body for performing a predetermined work to the ground, the soil quality information acquisition system being adapted for acquiring soil quality information being information about a soil quality on a worksite. The soil quality information acquisition system includes: a position information acquisition part that acquires position information about the working machine on the worksite; an acquisition time determination part that determines an acquisition time on the basis of soil quality relevant information being information related to a change in the soil quality on the worksite, and outputs an acquisition signal concerning the acquisition time; a soil quality information acquisition part that receives the acquisition signal output from the acquisition time determination part, and acquires the soil quality information in response to the acquisition signal; and a storage part that stores the position information acquired by the position information acquisition part and the soil quality information acquired by the soil quality information acquisition part in association with each other.

### Brief Description of Drawings

Fig. 1 is a side view of a working machine including a soil quality information acquisition system according to a first embodiment of the present invention.
Fig. 2 is a block diagram of the soil quality information acquisition system according to the first embodiment of the present invention.
Fig. 3 is a plan view of the working machine according to the first embodiment of the present invention to show a reference area for the working machine.
Fig. 4 is a side view of the working machine according to the first embodiment of the present invention to explain parameters for calculating an excavation radius for the working machine.
Fig. 5 is a flowchart showing initial setting by the soil quality information acquisition system according to the first embodiment of the present invention.
Fig. 6 is a flowchart showing soil quality information acquisition by the soil quality information acquisition system according to the first embodiment of the present invention.
Fig. 7 is a flowchart showing soil quality information acquisition by a soil quality information acquisition system according to a second embodiment of the present invention.

Fig. 8 is a flowchart showing soil quality information acquisition by a soil quality information acquisition system according to a third embodiment of the present invention.

Fig. 9 is a schematic diagram of a server included in a soil quality information acquisition system according to a fourth embodiment of the present invention.

Fig. 10 is a side of a working machine according to a fifth embodiment of the present invention to show a reference area for the working machine.

Fig. 11 is a plan view of a working attachment included in a working machine according to a sixth embodiment of the present invention.

## Description of Embodiments

Hereinafter, preferable embodiments of the present invention will be described with reference to the accompanying drawings.

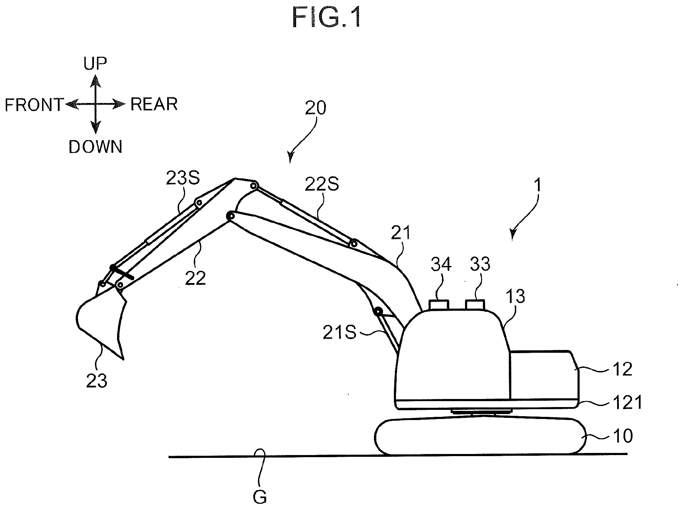

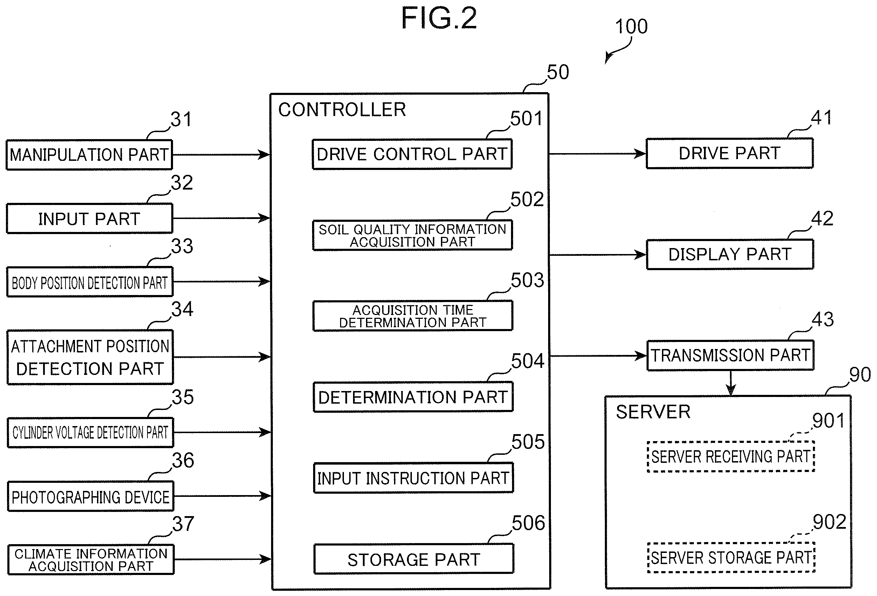

Fig. 1 is a side view of a hydraulic excavator 1 or working machine on which a soil quality information acquisition system 100 (Fig. 2) according to a first embodiment of the present invention is to be mounted.

The hydraulic excavator 1 includes a lower traveling body 10 travelable on a ground G (travelable surface), an upper slewing body 12 (upper body) slewably supported by the lower traveling body 10, and a working attachment 20 mounted on the upper slewing body 2. The lower traveling body 10 and the upper slewing body 12 constitute a machine body of the present invention.

The lower traveling body 10 is travelable on the ground. The lower traveling body 10 has a traveling part of a crawler type.

The upper slewing body 12 includes a slewing frame 121 supported on the lower traveling body 10, and a cab 13 mounted on the upper slewing frame 121. The cab 13 permits an operator to get therein, and includes various devices arranged to manipulate or operate the hydraulic excavator 1.

The working attachment 20 is attached to the upper slewing body 12 movably relative to the upper slewing body 12 for performing a predetermined work to the ground G. The working attachment 20 includes: a boom 21 connected to a front end of the slewing frame 121 rotatably in a tilting direction about a horizontal rotation central axis; an arm 22 connected to a distal end of the boom 21 rotatably about a horizontal rotation central axis; and a bucket 23 connected to a distal end of the arm 22 rotatably about a horizontal rotation central axis. In the embodiment, the rotation central axes of the boom 21, the arm 22, and the bucket 23 extend in parallel to one another. The boom 21 and the arm 22 constitute a tiltable body of the present invention, and the bucket 23 constitutes a working member of the present invention.

The working attachment 20 further includes: a boom cylinder 21S that extends and contracts to lower and raise the boom 21; an arm cylinder 22S that extends and contracts to rotate the arm 22; and a bucket cylinder 23S that extends and contracts to rotate the bucket 23. Each of the cylinders include a hydraulic cylinder.

The cab 13 is mounted on a front portion of the slewing frame 121 that is adjacent to the boom 21 in a width direction of the slewing frame 121 (on a left side of the boom 21 in the example shown in Fig. 1), and defines an operator compartment for maneuvering the hydraulic excavator 1. Specifically, the operator gives a manipulation in the cab 13 for each of the traveling of the lower traveling body 10, the slewing of the upper slewing body 12, and the activation of the working attachment 20.

Fig. 2 is a block diagram of the hydraulic excavator 1 including the soil quality information acquisition system 100, according to the first embodiment. The soil quality information acquisition system 100 acquires soil quality information being information about a soil quality on a worksite. The hydraulic excavator 1 further includes: a manipulation part 31: an input part 32; a body position detection part 33 (position information acquisition part); an attachment position detection part 34 (distance detection part); a cylinder pressure detection part 35; a photographing device 36; a climate information acquisition part 37; a drive part 41; a display part 42; and a transmission part 43.

The manipulation part 31 is arranged in the cab 13 to be manipulated by the operator. Specifically, the manipulation part 31 receives a manipulation to operate the hydraulic excavator 1. The operation by the manipulation includes the traveling of the lower traveling body 10, the slewing of the upper slewing body 12, and the driving of the working attachment 20 (the boom 21, the arm 22, and the bucket 23).

The input part 32 is arranged in the cab 13 to receive an input of each kind of information. Examples of the input part 32 include various kinds of input buttons, switches, and a touch screen included in the display part 42 to be described later. In particular, the input part 32 is configured to receive an input of a distance from the upper slewing body 12 (a slewing central axis CL) to a distal end (bucket distal end 23A) of the working attachment 20, the distance being included in information to be referred to in soil quality information acquisition to be described later.

The body position detection part 33 acquires position information about the hydraulic excavator 1 on the worksite. For instance, the body position detection part 33 acquires body coordinate information being information related to an absolute coordinate of a body reference point on the worksite, the body reference point being provided for the upper slewing body 12 in advance. The body position detection part 33 that creates the body reference point is located on a top of the cab 13 and serves as a mobile station of the GNSS. The soil quality information acquisition system 100 has a reference station (not shown) of the GNSS (Global Navigation Satellite System) to acquire the body coordinate information. The reference station of the GNSS is arranged on the worksite, or arranged at a position closest to the worksite. The adoptable GNSS includes a publicly known GPS (Global Positioning System), and additionally, a satellite positioning, navigation and timing system, such as the GLONASS (Global Navigation satellite system), the Galileo, and the QZSS (Quasi-Zenith Satellite System).

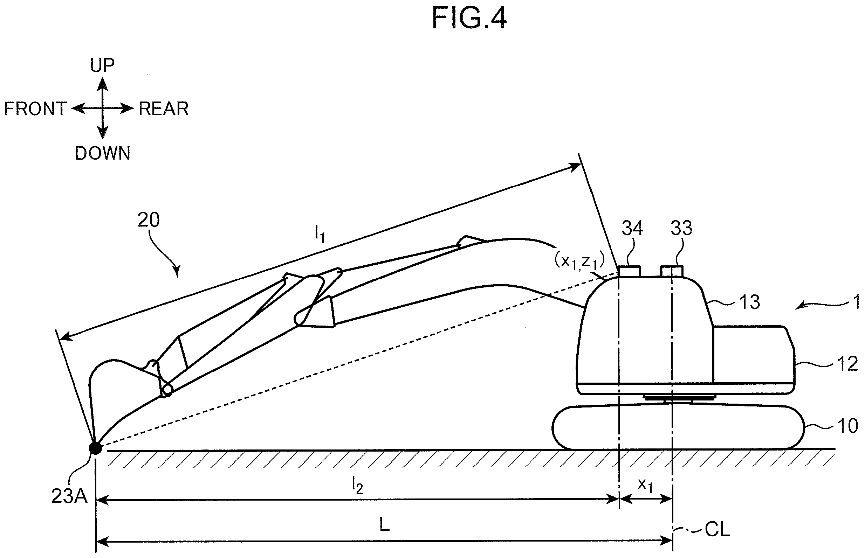

The attachment position detection part 34 is arranged on a front end of the top of the cab 13. For instance, the attachment position detection part 34 includes a LiDAR (Light Detection And Ranging) sensor. The attachment position detection part 34 can detect a distance L (Fig. 4) from the upper slewing body 12 to the bucket distal end 23A of the bucket 23. The attachment position detection part 34 may include a TOF (Time Of Flight) sensor or a stereo camera.

The cylinder pressure detection part 35 is arranged in a hydraulic circuit for driving the working attachment 20, and detects a cylinder pressure of each of the boom cylinder 21S, the arm cylinder 22S, and the bucket cylinder 23S. A soil quality information acquisition part 502 to be described later refers to each cylinder pressure detected by the cylinder pressure detection part 35 to estimate the soil quality (ground hardness) on the worksite.

The photographing device 36 is arranged at a leading end of the cab 13. The photographing device 36 captures an image of the ground around the bucket 23 of the working attachment 20. The soil quality information acquisition part 502 refers to the image captured by the photographing device 36 for estimation of the soil quality on the worksite. The attachment position detection part 34 may serve as the photographing device 36.

The climate information acquisition part 37 is configured to acquire climate information for the worksite. As an example, the climate information acquisition part 37 is attached to the hydraulic excavator 1, and includes a thermo-hygrometer that acquires temperature and humidity information therefor around. As another example, the climate information acquisition part 37 is connected to the Internet or the like to acquire climate information for the worksite on the basis of forecast information provided by, for example, a meteorological agency. The climate information includes a predicted value of a rainfall amount, and temperatures and humidities in morning, daytime, and nighttime. The climate information acquisition part 37 may acquire, as the climate information, an accumulative rain fall amount measured by a rain gauge provided on the worksite in a specific period.

The drive part 41 is configured to drive various structures of the hydraulic excavator 1, that is, drive the lower traveling body 10, the upper slewing body 12, and the working attachment 20 each operated by the manipulation part 31. The drive part 41 includes a hydraulic circuit, such as a hydraulic pump and a hydraulic motor.

The display part 42 is arranged in the cab 13 to display various kinds of information and notify the operator of the information through the displaying. The information includes soil quality information and position information about the hydraulic excavator 1, the quality information and the position information being acquired by the soil quality information acquisition system 100.

The transmission part 43 transmits, to a server 90 to be described later, the position information about the hydraulic excavator 1 acquired by the body position detection part 33 and the soil quality information for the worksite acquired by the soil quality information acquisition part 502.

The controller 50 includes a CPU (Central Processing Unit), a ROM (Read Only Memory) which stores a control program, and a RAM (Random Access Memory) for use as a work area of the CPU. As illustrated in Fig. 2, the manipulation part 31, the input part 32, the body position detection part 33, the attachment position detection part 34, the cylinder pressure detection part 35, the photographing device 36, the climate information acquisition part 37, the drive part 41, the display part 42, and the transmission part 43 are connected to the controller 50. The controller 50 functions to include a drive control part 501, the soil quality information acquisition part 502, an acquisition time determination part 503, a determination part 504, an input instruction part 505, and a storage part 506 when the CPU executes the control program stored in the ROM. The functional parts have no entities, and respectively correspond to units of functions to be executed by the control program. Here, an entirety or a part of the controller 50 may not be limitedly provided in the hydraulic excavator 1, and may be provided at a different position other than the position in the hydraulic excavator 1 for a remote control of the hydraulic excavator 1. Further, the control program may be transmitted from a server or management device at a remote location or a cloud to the controller 50 in the hydraulic excavator 1 to be executed, or the control program may be executed on the server or the cloud and each kind of generated instruction signal may be transmitted to the hydraulic excavator 1.

The drive control part 501 inputs a drive instruction signal to the drive part 41 in accordance with a content of a manipulation received by the manipulation part 31. As a result, the operation of each of the lower traveling body 10, the upper slewing body 12, and the working attachment 20 is controlled.

The soil quality information acquisition part 502 receives an acquisition signal output from the acquisition time determination part 503, and acquires the soil quality information for the worksite in response to the acquisition signal. The soil quality information acquisition part 502 may adopt a publicly known way to acquire the soil quality information. For instance, the soil quality information acquisition part 502 can acquire the soil quality information (ground hardness) about the ground G in accordance with a counterforce accompanied by contact of the bucket distal end 23A of the working attachment 20 with the ground G. The counterforce can be estimated from each cylinder pressure detected by the cylinder pressure detection part 35. At this time, the working attachment 20 may have a preset reference posture. By contrast, when the counterforce is detected in a specific posture of the working attachment, the detected counterforce may be corrected depending on a posture (rotation angle, ground angle) of each of the boom 21, the arm 22 and the bucket 23.

The acquisition time determination part 503 determines an acquisition time for acquiring soil quality information on the basis of soil quality relevant information being information related to a change in the soil quality on the worksite, and outputs an acquisition signal concerning the acquisition time.

The determination part 504 executes various kinds of determination in the soil quality information acquisition executed by the soil quality information acquisition part 502 and the acquisition time determination part 503.

For transmission of the acquired soil quality information to the server 90, the input instruction part 505 inputs an instruction signal corresponding to the soil quality information to the transmission part 43.

The storage part 506 stores the position information about the hydraulic excavator 1 acquired by the body position detection part 33 and the soil quality information acquired by the soil quality information acquisition part 502 in association with each other.

In the configuration, the input part 32, the body position detection part 33, the attachment position detection part 34, the cylinder pressure detection part 35, the photographing device 36, the climate information acquisition part 37, the transmission part 43, and the controller 50 constitute a part of the soil quality information acquisition system 100. The soil quality information acquisition system 100 further includes the server 90 or management device.

The server 90 is located away from the worksite where the hydraulic excavator 1 performs a work, e.g., arranged in a data management center or a remote control center that totally manages works of a plurality of hydraulic excavators 1. The server 90 has a server receiving part 901 and a server storage part 902.

The server receiving part 901 is located away from the transmission part 43, receives the position information and the soil quality information transmitted from the transmission part 43, and inputs and stores the position information and the soil quality information in the server storage part 902.

The server storage part 902 stores the position information and the soil quality information received by the transmission part 43 in association with each other.

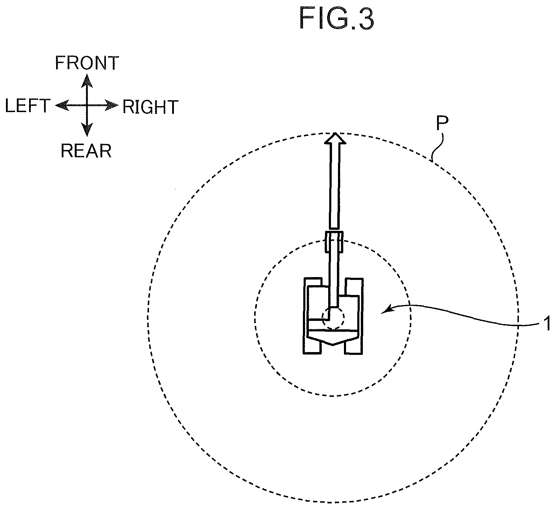

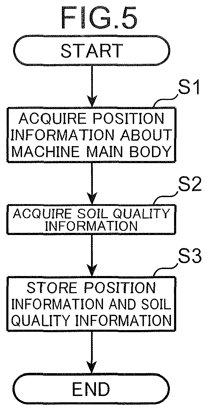

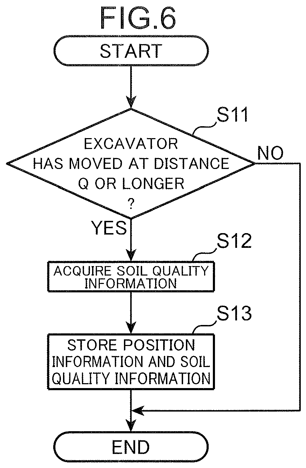

Fig. 3 is a plan view of the hydraulic excavator 1 according to the first embodiment to show a reference area for the excavator. Fig. 4 is a side view of the hydraulic excavator 1 according to the embodiment to explain parameters for calculating an attachment length or an excavation radius for the excavator. Fig. 5 is a flowchart showing initial setting by the soil quality information acquisition system 100. Fig. 6 is a flowchart showing soil quality information acquisition by the soil quality information acquisition system 100.

In the embodiment, the acquisition time determination part 503 of the soil quality information acquisition system 100 determines an appropriate acquisition time for acquiring soil quality information, and the soil quality information acquisition part 502 acquires the soil quality information at the determined time.

The acquisition time determination part 503 sets a reference area P (also referred to as an applicable area of the soil quality information) virtually estimated to have a specific soil quality on the worksite (Fig. 3), and determines the acquisition time on the basis of a relative position of the hydraulic excavator 1 to the reference area P, the relative position being included in the soil quality relevant information. In the embodiment, as illustrated in Fig. 3, the acquisition time determination part 503 sets, as the reference area P, an area defined by a reference circle having a predetermined radius from the slewing central axis CL of the upper slewing body 12 serving as the center thereof in a plan view at a predetermined reference time (at initial setting). Specifically, the soil quality information acquisition is executed by the soil quality information acquisition system 100 on the basis of a technical concept of a requirement for acquisition of additional soil quality information due to a difference in soil quality outside the reference area P on the premise that the soil quality is uniform within the reference area P shown in Fig. 3.

Referring to Fig. 4, the attachment position detection part 34 detects a maximum length 11 of the working attachment 20 from a reference point (x1, z1) set at the upper end of the cab 13. The x-coordinate of the reference point corresponds to a distance from the slewing central axis CL to the reference point in a horizontal direction. The z-coordinate of the reference point indicates, for example, a height from the ground. As a result, the distance L (maximum excavation radius) from the slewing central axis CL to the bucket distal end 23A is calculated from Equation 1 and Equation 2 shown below.

Formula 1

$$l_2 = \sqrt{l_1{}^2 - z_1{}^2} \quad \text{...Equation 1}$$

Formula 2

$$L = l_2 + x_1 = \sqrt{l_1{}^2 - z_1{}^2} + x_1 \quad \text{...Equation 2}$$

Fig. 3 shows: a circle (inner circle) having a radius of the distance L and drawn by the bucket distal end 23A of the working attachment 20 at present in slewing of the upper slewing body 12 about the slewing central axis CL; and a circle having a radius of a distance 2L surrounding the inner circle. Specifically, the reference area P shown in Fig. 3 represents the circle having the radius of the distance 2L from the slewing central axis CL serving as the center. The radius of the reference area P may be set to a specific value exceeding the distance L.

The reference area P may be set suitably for a purpose of a working machine or construction machine represented by the hydraulic excavator 1 at a time of transportation from a factory. The operator may input the setting from the input part 32 on the worksite.

In particular, a dimension (radius) of the reference area P may be selectively set from the following ways: (i) adopting a value stored in advance in association with the reference area P (applicable area) at the transportation from the factory; (ii) automatically setting a radius suitable for an appropriate length of the working attachment 20 selected by the operator from various lengths stored in the storage part 506 and displayed on the display part 42; and (iii) inputting machine specification information necessary for calculating a radius of the reference area P from the input part 32 by the operator, calculating the distance L by the soil quality information acquisition part 502 when the working attachment 20 has a preset reference posture, and defining the distance 2L as the radius of the reference area P. The machine specification information includes information about the length of each of the boom 21, the arm 22, and the bucket 23. The reference posture includes a preset posture of each of the boom 21, the arm 22, and the bucket 23 to the upper slewing body 12.

Referring to Fig. 5, the soil quality information acquisition system 100 executes initial setting in response to a predetermined work start signal input by the operator from the input part 32 to start a specific work on a worksite. At this time, the body position detection part 33 acquires position information about the hydraulic excavator 1 on the worksite (step S1). Next, the drive control part 501 drives the working attachment 20 to bring the bucket distal end 23A into contact with the ground G at a predetermined speed. The soil quality information acquisition part 502 acquires soil quality information on the basis of a cylinder pressure detected by the cylinder pressure detection part 35 at this time (step S2). Here, the soil quality information may be estimated on the basis of an image of the ground G captured by the photographing device 36 as described above. Subsequently, the storage part 506 stores the acquired position information and the soil quality information in association with each other (step S3).

Referring to Fig. 6, when the hydraulic excavator 1 starts the work, the determination part 504 determines whether the hydraulic excavator 1 moves at a distance Q or longer in comparison with the initial setting with reference to the position information detected by the body position detection part 33 (step S11). For example, the distance Q is set to the distance L. When the hydraulic excavator 1 moves at the distance Q or longer (YES in step S11), the acquisition time determination part 503 inputs an acquisition signal to the soil quality information acquisition part 502 to acquire new soil quality information. Consequently, the soil quality information acquisition part 502 acquires the soil quality information (step S12). Then, the storage part 506 stores the acquired soil quality information and current position information about the hydraulic excavator 1 in association with each other (step S13).

Table 1 shows a relationship between the position information and the soil quality information stored in the storage part 506.

[Table 1]

| Position information | Soil quality information |
|:---:|:---:|
| A | a |
| B | b |
| C | b |
| D | c |
| • | • |
| • | • |
| • | • |

As shown in the table, the storage part 506 stores a plurality of pieces of information (a combination of the soil quality information and the position information) for a current worksite. In use of only the newest information, the information at the initial setting may be overwritten with the newest information in step S13.

While the storage part 506 stores the information, the input instruction part 505 may display an input signal corresponding to the information on the display part 42 to notify the operator of the soil quality information therearound. Further, the server storage part 902 may store the same information via the server receiving part 901 in response to an input of the input signal by the input instruction part 505 to the transmission part 43.

When the hydraulic excavator 1 does not move at the distance Q or longer (NO in step S11) in step S11 in Fig. 6, the soil quality information acquisition part 502 finishes the process in Fig. 6 without acquiring new soil quality information. The process shown in Fig. 6 is repetitively executed in the work of the hydraulic excavator 1.

As described heretofore, in the embodiment, the acquisition time determination part 503 can determine an acquisition time on the basis of the soil quality relevant information being information related to a change in the soil quality on the worksite, and the soil quality information acquisition part 502 can acquire the soil quality information at the determined acquisition time. The soil quality information is acquired at the appropriate time in accordance with a possibility of a change in the soil quality information around the hydraulic excavator 1, and thus, acquisition of excessive information and acquisition of insufficient information are preventable. This results in allowing the storage part 506 to have a smaller required storage capacity than a certain storage part which needs to store periodically acquired soil quality information together with position information.

In the embodiment, the acquisition time determination part 503 sets the reference area P virtually estimated to have a specific soil quality on the worksite, and determines the acquisition time on the basis of a relative position of the hydraulic excavator 1 to the reference area, the relative position being included in the soil quality relevant information. This configuration enables effective acquisition and storage of the soil quality information at a more appropriate time by setting the reference area P depending on a state of the worksite, and a size and a purpose of the hydraulic excavator.

In particular, in the embodiment, the acquisition time determination part 503 sets, as the reference area P, an area defined by a reference circle having a predetermined radius from the upper slewing body 12 (slewing central axis CL) serving as the center in a plan view at a predetermined reference time (at initial setting). This configuration enables determination of the acquisition time of the soil quality information on the basis of a relative position of the hydraulic excavator 1 to the reference circle with a focus on a similarity in soil quality in a predetermined area in a plan view on the worksite. This succeeds in preventing frequent acquisition of soil quality information in an area having the same soil quality.

Further, in the embodiment, the acquisition time determination part 503 outputs an acquisition signal concerning the acquisition time when the hydraulic excavator 1 goes beyond a region enclosed by a boundary circle (having a radius of the distance L) falling within the reference circle and being concentric with the reference circle in traveling of the lower traveling body 10 (traveling part). This configuration enables acquisition of new soil quality information when the hydraulic excavator 1 goes beyond a preset boundary circle, and thus achieves acquisition of appropriate soil quality information for a work position even when the hydraulic excavator 1 performs the work as moving outward from the reference circle.

The acquisition time determination part 503 may output the acquisition signal when the working attachment 20 extends beyond the reference circle (having the radius of the distance 2L). This configuration enables acquisition of new soil quality information when the working attachment 20 extends beyond a reference circle, and thus achieves acquisition of appropriate soil quality information for a work position even when the hydraulic excavator 1 performs the work as moving outward from the reference circle.

By contrast, the acquisition time determination part 503 may set the radius of the reference circle on the basis of a distance between the upper slewing body 12 and the bucket distal end 23A detected by the attachment position detection part 34 or the photographing device 36 (distance detection part). For instance, a value twice as large as the distance may be defined as the radius of the reference circle. This configuration enables appropriate setting of the reference circle depending on an actual length of the working attachment 20 detected by the distance detection part regardless of the specification of the working attachment 20 attached to the upper slewing body 12. The radius of the reference circle may be set to a specific value exceeding the distance.

The acquisition time determination part 503 may further set the radius of the reference circle on the basis of a distance between the upper slewing body 12 and the bucket distal end 23A input to the input part 32. For instance, a value twice as large as the distance may be defined as the radius of the reference circle in this case as well. This configuration also enables appropriate setting of the reference circle depending on the length of the working attachment 20 input by the operator regardless of the specification of the working attachment 20 attached to the upper slewing body 12. The radius of the reference circle may be set to a specific value exceeding the distance in this case as well.

Next, a second embodiment of the present invention will be described. Here, in the embodiment, differences from the preceding first embodiment will be described and matters in common therewith will be omitted. The same way is applied to the embodiments to be described below.

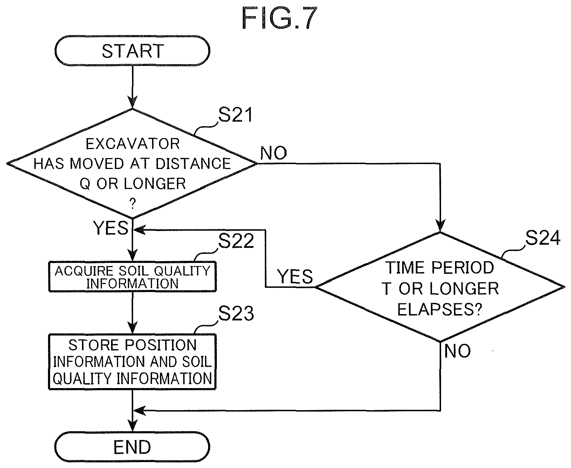

Fig. 7 is a flowchart showing soil quality information acquisition by a soil quality information acquisition system 100 according to the embodiment. In the embodiment, steps 21, S22, and S23 are sequentially executed in the same manner as steps S11, S12, and S13 in the preceding first embodiment (Fig. 6). Further, in the embodiment, when a hydraulic excavator 1 does not move at a distance Q or longer in step S21 (NO in step S21), a determination part 504 determines whether a time period T or longer elapses from a previous acquisition time of soil quality information (step S24). Here, when the time period T or longer elapses, new soil quality information is acquired in step S22, and a storage part 506 stores the soil quality information and position information in association with each other in step S23. As a result, a plurality of pieces of soil quality information is acquirable with a variable based on a time difference under a condition of no large movement of the hydraulic excavator 1, in other words, under a condition of almost the same position of the hydraulic excavator on a worksite.

When the time period T or longer does not elapse in step S24 (NO in step S24), a soil quality information acquisition part 502 finishes the process in Fig. 7 without acquiring new soil quality information.

The embodiment considers a possibility of a change in a soil quality attributed to a change in a water amount in the ground due to a temperature change difference between morning and daytime. Specifically, even in no large movement of the hydraulic excavator 1, the soil quality information acquisition part 502 acquires new soil quality information after a lapse of a predetermined time period, e.g., four hours. Here, the predetermined time period is not limited to four hours, and may be set in consideration of, for example a climate on the worksite.

In another embodiment, the predetermined time period may be set at transportation of the hydraulic excavator 1 from a factory in consideration of a climate at a destination of the hydraulic excavator. Alternatively, daytime temperature difference information may be acquired from climate information on a region including a worksite in accordance with position information about the hydraulic excavator 1. Here, the predetermined time period may be decreased when the temperature difference is large, and the predetermined time period may be increased when the temperature difference is small.

Consequently, in the embodiment, an acquisition time determination part 503 determines an acquisition time on the basis of an elapsed time period from a previous acquisition time of soil quality information acquired by the soil quality information acquisition part 502, the elapsed time period being included in soil quality relevant information. In this manner, a change in the soil quality attributed to a time transition is acquirable at an appropriate time even in performance of a work by the hydraulic excavator 1 without a change in the position of the machine body thereof.

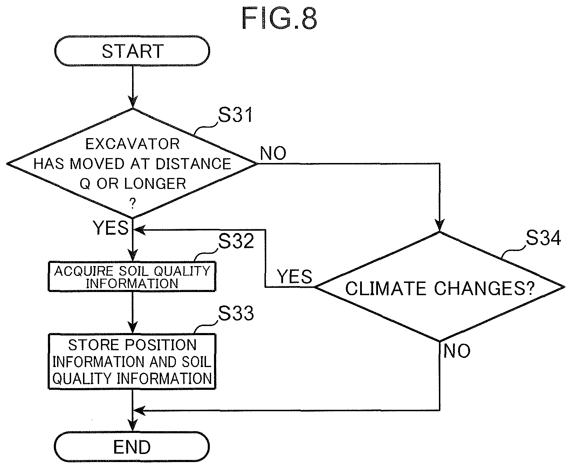

Next, a third embodiment of the present invention will be described. Fig. 8 is a flowchart showing soil quality information acquisition by a soil quality information acquisition system 100 according to the embodiment.

In the embodiment, steps 31, S32, and S33 are sequentially executed in the same manner as steps S11, S12, and S13 in the preceding first embodiment (Fig. 6). Besides, in the embodiment, when a hydraulic excavator 1 does not move by a distance Q or longer in step S31 (NO in step S31), a determination part 504 determines whether a climate changes in comparison with a previous acquisition time of soil quality information (step S34). Acquisition information acquired by a climate information acquisition part 37 as described above is referred to in the determination. Here, when the climate on a worksite changes or is estimated to change, new soil quality information is acquired in step S32, and a storage part 506 stores the soil quality information and position information in association with each other in step S33. As a result, a plurality of pieces of soil quality information is acquirable with a variable based on a difference in climate information under a condition of no large movement of the hydraulic excavator 1, in other words, under a condition of almost the same position of the hydraulic excavator on the worksite.

When the climate therearound does not change in step S34 (NO in step S34), a soil quality information acquisition part 502 finishes the process in Fig. 8 without acquiring new soil quality information.

Consequently, in the embodiment, an acquisition time determination part 503 determines an acquisition time on the basis of climate information acquired by the climate information acquisition part 37, the climate information being included in soil quality relevant information. This configuration enables acquisition of a change in the soil quality attributed to the climate at an appropriate time even in performance of a work by the hydraulic excavator 1 without a change in the position of the machine body thereof.

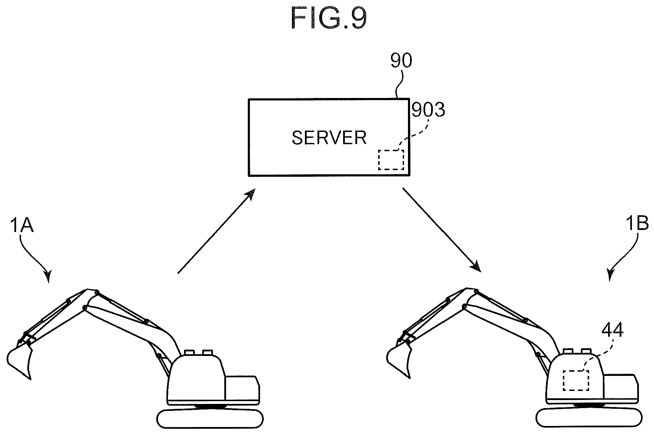

Next, a fourth embodiment of the present invention will be described. Fig. 9 is a schematic diagram of a server 90 included in a soil quality information acquisition system 100 according to the embodiment. In the preceding first embodiment, in addition to the storage part 506 of the controller 50, the server 90 also stores the soil quality information and the position information about the hydraulic excavator 1 in association with each other, the soil quality information and the position information being acquired in the hydraulic excavator 1 (corresponding to a hydraulic excavator 1A illustrated in Fig. 9). In this case, the server storage part 902 at a remote location stores the position information and the soil quality information acquired on the worksite to achieve accumulation of the soil quality information. As a result, in a subsequent work, the hydraulic excavator 1 is automatically controllable with effective use of the information.

By contrast, in the embodiment, a server 90 further has an auxiliary transmission part 903. The auxiliary transmission part 903 transmits position information and soil quality information acquired by a hydraulic excavator 1A and stored in a server storage part 902 to a hydraulic excavator 1B (another working machine) on a worksite. A soil quality information acquisition system 100 further includes an auxiliary receiving part 44. The auxiliary receiving part 44 is arranged at the hydraulic excavator 1B, receives the position information and the soil quality information transmitted from the auxiliary transmission part 903, and causes a storage part 506 included in the hydraulic excavator 1B to store the information by inputting the information thereinto.

As described above, in the embodiment, transmission of the soil quality information acquired by the one hydraulic excavator 1A to another hydraulic excavator 1B on the same worksite leads to effective utilization and sharing of the soil quality information among a plurality of hydraulic excavators. In particular, a frequency of acquiring soil quality information in the hydraulic excavator 1B is reducible.

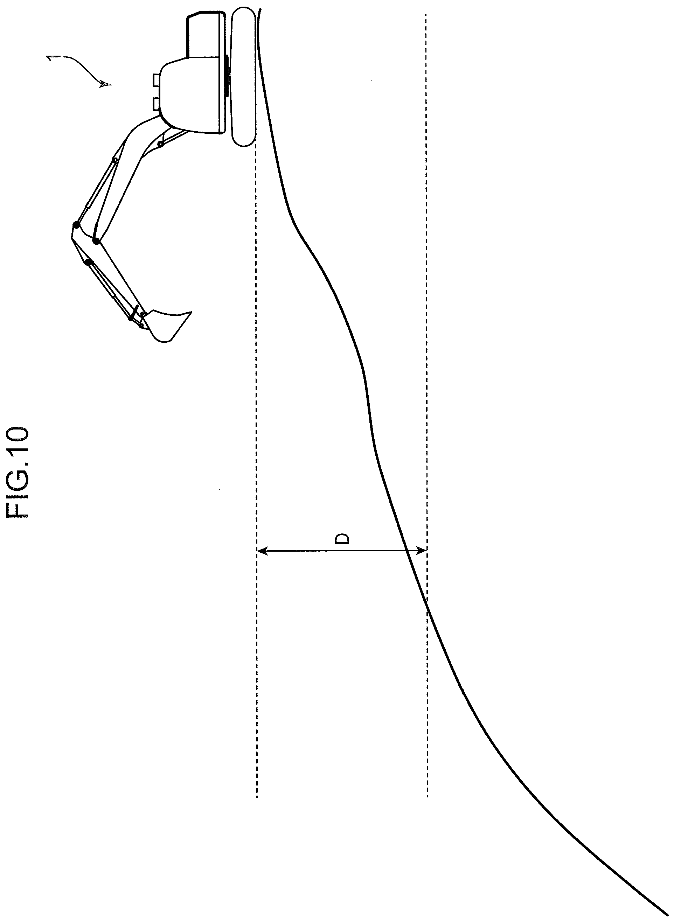

Next, a fifth embodiment of the present invention will be described. Fig. 10 is a side view of a hydraulic excavator 1 according to the embodiment to show a reference area for the excavator. In the embodiment, an acquisition time determination part 503 sets, as a reference area D, a range from a lower traveling body 10 to a predetermined depth under the ground at a predetermined reference time (initial setting). The embodiment is based on the presumption that the soil quality is almost uniform in the range to the predetermined depth under the ground.

This embodiment achieves determination of an acquisition time of soil quality information on the basis of a relative position of the working machine to the reference area with a focus on a similarity in soil quality in a predetermined range in a depth direction under the ground on the worksite. This succeeds in preventing frequent acquisition of soil quality information in a range in a depth direction having the same soil quality.

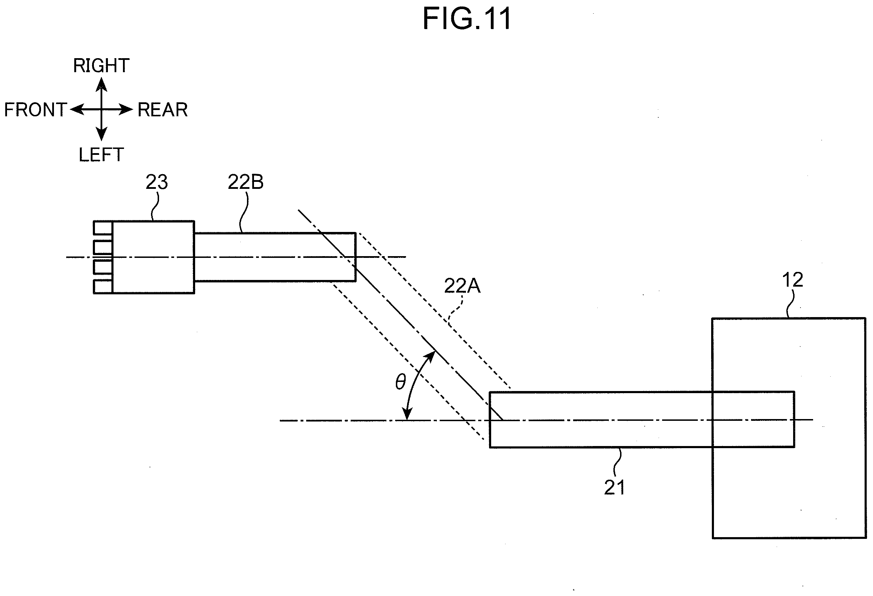

Next, a sixth embodiment of the present invention will be described. Fig. 11 is a plan view of a working attachment 20 included in a hydraulic excavator 1 according to the embodiment. In the preceding first embodiment, each of the boom 21, the arm 22, and the bucket 23 rotates or swings about the horizontal rotation central axis. In the embodiment, the working attachment 20 includes a swing boom 22A, and the swing boom 22A connects a boom 21 and an arm 22B to each other. The swing boom is swingable about a rotational central axis extending in a longitudinal direction of the boom 21 (called an offset boom manner).

Specifically, in the embodiment, an angle $\theta$ between the boom 21 and the swing boom 22A in a plan view is defined as a variable. Although Fig. 11 shows a state of the swing boom 22A swinging to right, the swing boom 22A is swingable to left as well. When an offset amount (swing angle) to each of the left and the right is defined as maximum, a reference area P is desirably set in consideration of a decrease in a bottom surface maximum excavation radius. Moreover, in the embodiment, a relative position of a bucket 23 (bucket distal end 23A) to an upper slewing body 12 may be geometrically calculated from information about a length and a relative angle of the boom 21 to the upper slewing body 12, a length and a relative angle of the swing boom 22A to the boom 21, a length and a relative angle of the arm 22B to the swing boom 22A, and a length and a relative angle of the bucket 23 to the arm 22B.

As described heretofore, even when the working attachment 20 has a distinctive configuration, the embodiment achieves acquisition of soil quality information at an appropriate time by setting a reference area P suitably for the configuration.

Heretofore, a soil quality information acquisition system and a working machine including the system according to each of the embodiments have been described. The present invention should not be limited to the embodiments. The present invention can cover, for example, the following modified embodiments.

Although the hydraulic excavator 1 is used to describe a working machine in each embodiment, the present invention is not limited thereto. The working machine according to the present invention may have a configuration other than that of the hydraulic excavator. In particular, a working member to be provided to the distal end of the working attachment 20 is not limited to the bucket 23. Further, the upper body according to the present invention may not slew about the lower traveling body 10. In this case, the front-rear direction of the upper body agrees with the front-rear direction of the lower traveling body 10.

The present invention provides a soil quality information acquisition system for use in a working machine including: a machine body having a traveling part travelable on a ground; and a working attachment attached to the machine body movably relative to the machine body for performing a predetermined work to the ground, the soil quality information acquisition system being adapted for acquiring soil quality information being information about a soil quality on a worksite. The soil quality information acquisition system includes: a position information acquisition part that acquires position information about the working machine on the worksite; an acquisition time determination part that determines an acquisition time on the basis of soil quality relevant information being information related to a change in the soil quality on the worksite, and outputs an acquisition signal concerning the acquisition time; a soil quality information acquisition part that receives the acquisition signal output from the acquisition time determination part, and acquires the soil quality information in response to the acquisition signal; and a storage part that stores the position information acquired by the position information acquisition part and the soil quality information acquired by the soil quality information acquisition part in association with each other.

According to the configuration, the acquisition time determination part can determine an acquisition time on the basis of the soil quality relevant information being information related to a change in the soil quality on the worksite, and the soil quality information acquisition part can acquire the soil quality information at the determined acquisition time. The soil quality information is acquired at the appropriate time in accordance with a possibility of the change in the soil quality information around the working machine, and thus, acquisition of excessive information and acquisition of insufficient information are preventable. This results in allowing the storage part to have a smaller required storage capacity than a storage part which needs to store periodically acquired soil quality information together with position information.

In the configuration, the acquisition time determination part desirably sets a reference area virtually estimated to have a specific soil quality on the worksite, and determines the acquisition time on the basis of a relative position of the working machine to the reference area, the relative position being included in the soil quality relevant information.

This configuration enables effective acquisition and storage of the soil quality information at a more appropriate time on the basis of the relative position of the working machine to the reference area.

In the configuration, the acquisition time determination part desirably sets, as the reference area, an area defined by a reference circle having a predetermined radius from the center of the machine body in a plan view at a predetermined reference time.

This configuration enables determination of the acquisition time of the soil quality information on the basis of a relative position of the working machine to the reference circle with a focus on a similarity in soil quality in a predetermined area in a plan view on the worksite. This succeeds in preventing frequent acquisition of soil quality information in an area having the same soil quality.

In the configuration, the acquisition time determination part desirably outputs the acquisition signal when the working machine goes beyond a boundary circle falling within the reference circle and being concentric with the reference circle in traveling of the traveling part.

This configuration enables acquisition of new soil quality information when the working machine goes beyond a preset boundary circle, and thus achieves acquisition of appropriate soil quality information for a work position even when the working machine performs the work as moving outward from the reference circle.

In the configuration, the acquisition time determination part desirably outputs the acquisition signal when the working attachment extends beyond the reference circle.

This configuration enables acquisition of new soil quality information when the working attachment extends beyond a preset reference circle, and thus achieves acquisition of appropriate soil quality information for a work position even when the working machine performs the work as moving outward from the reference circle.

In the configuration, the acquisition time determination part desirably sets, as the reference area, a range from the machine body to a predetermined depth under the ground at a predetermined reference time.

This configuration enables determination of an acquisition time of soil quality information on the basis of a relative position of the working machine to the reference area with a focus on a similarity in soil quality in a predetermined range in a depth direction under the ground on the worksite. This succeeds in preventing frequent acquisition of soil quality information in a range in a depth direction having the same soil quality.

In the configuration, the acquisition time determination part desirably determines the acquisition time on the basis of an elapsed time period from a previous acquisition time of soil quality information by the soil quality information acquisition part, the elapsed time period being included in the soil quality relevant information.

This configuration enables acquisition of the soil quality information at an appropriate time in consideration of a change in the soil quality attributed to a time transition even in performance of a work without a change in the position of the machine body.

This configuration desirably further includes: a climate information acquisition part configured to acquire climate information for the worksite. The acquisition time determination part desirably determines the acquisition time on the basis of the climate information acquired by the climate information acquisition part, the climate information being included in the soil quality relevant information.

This configuration enables acquisition of the soil quality information at an appropriate time in consideration of a change in the soil quality attributed to a climate even in performance of a work without a change in the position of the machine body.

In the configuration, the storage part is located away from the working machine. This configuration desirably further includes: a transmission part that transmits the position information acquired by the position information acquisition part and the soil quality information acquired by the soil quality information acquisition part; and a receiving part that is located away from the transmission part, and receives the position information and the soil quality information transmitted from the transmission part and inputs the position information and the soil quality information into the storage part.

This configuration causes the storage part at a remote location to store the position information and the soil quality information acquired on the worksite to achieve accumulation of the soil quality information.

This configuration desirably further includes: an auxiliary transmission part that transmits the position information and the soil quality information stored in the storage art to another working machine on the worksite; and an auxiliary receiving part that is arranged at the another working machine, and receives the position information and the soil quality information transmitted from the auxiliary transmission part.

This configuration is configured to transmit the soil quality information acquired by one working machine to another working machine on the same worksite to enable effective utilization and sharing of the soil quality information among a plurality of hydraulic excavators.

The present invention provides a working machine. The working machine includes: a machine body having a traveling part travelable on a ground; a working attachment supported by the machine body movably relative to the machine body for performing a predetermined work to the ground; and any soil quality information acquisition system described above.

According to the configuration, in the working machine, the acquisition time determination part can determine an acquisition time on the basis of the soil quality relevant information being information related to a change in the soil quality on the worksite, and the soil quality information acquisition part can acquire the soil quality information at the determined acquisition time. The soil quality information is acquired at the appropriate time in accordance with a possibility of the change in the soil quality information around the working machine, and thus, acquisition of excessive information and acquisition of insufficient information are preventable. This results in allowing the storage part of the working machine to have a smaller required storage capacity than a storage part which needs to store periodically acquired soil quality information together with position information.

The present invention provides a soil quality information acquisition system that enables acquisition of soil quality information at an appropriate time on a worksite, and a working machine including the system.

**Claims**

1. A soil quality information acquisition system (100) for use in a working machine (1) including: a machine body (10, 12) having a traveling part travelable on a ground; and a working attachment (20) attached to the machine body (10, 12) movably relative to the machine body (10, 12) for performing a predetermined work to the ground, the soil quality information acquisition system (100) being adapted for acquiring soil quality information being information about a soil quality on a worksite, the soil quality information acquisition system comprising:

   a position information acquisition part (33) that acquires position information about the working machine on the worksite;
   an acquisition time determination part (503) that determines an acquisition time on the basis of soil quality relevant information being information related to a change in the soil quality on the worksite, and outputs an acquisition signal concerning the acquisition time;
   a soil quality information acquisition part (502) that receives the acquisition signal output from the acquisition time determination part (503), and acquires the soil quality information in response to the acquisition signal; and
   a storage part (506) that stores the position information acquired by the position information acquisition part (33) and the soil quality information acquired by the soil quality information acquisition part (502) in association with each other.

2. The soil quality information acquisition system (100) according to claim 1, wherein the acquisition time determination part (503) sets a reference area virtually estimated to have a specific soil quality on the worksite, and determines the acquisition time on the basis of a relative position of the working machine (1) to the reference area, the relative position being included in the soil quality relevant information.

3. The soil quality information acquisition system (100) according to claim 2, wherein the acquisition time determination part (503) sets, as the reference area, an area defined by a reference circle having a predetermined radius from the center of the machine body (10, 12) in a plan view at a predetermined reference time.

4. The soil quality information acquisition system (100) according to claim 3, wherein the acquisition time determination part (503) outputs the acquisition signal when the working machine (1) goes beyond a boundary circle falling within the reference circle and being concentric with the reference circle in traveling of the traveling part.

5. The soil quality information acquisition system (100) according to claim 3, wherein the acquisition time determination part (503) outputs the acquisition signal when the working attachment (20) extends beyond the reference circle.

6. The soil quality information acquisition system (100) according to claim 2, wherein the acquisition time determination part (503) sets, as the reference area, a range from the machine body (10, 12) to a predetermined depth under the ground at a predetermined reference time.

7. The soil quality information acquisition system (100) according to any one of claims 1 to 6, wherein the acquisition time determination part (503) determines the acquisition time on the basis of an elapsed time period from a previous acquisition time of soil quality information by the soil quality information acquisition part (502), the elapsed time period being included in the soil quality relevant information.

8. The soil quality information acquisition system (100) according to any one of claims 1 to 7, further comprising a climate information acquisition part configured to acquire climate information for the worksite, wherein
the acquisition time determination part (503) determines the acquisition time on the basis of the climate information acquired by the climate information acquisition part, the climate information being included in the soil quality relevant information.

9. The soil quality information acquisition system (100) according to any one of claims 1 to 8, wherein the storage part (506) is located away from the working machine (1), the soil quality information acquisition system (100) further comprising:

   a transmission part (43) that transmits the position information acquired by the position information acquisition part (33) and the soil quality information acquired by the soil quality information acquisition part (502); and

a receiving part (44) that is located away from the transmission part (43), and receives the position information and the soil quality information transmitted from the transmission part (43) and inputs the position information and the soil quality information into the storage part (506).

10. The soil quality information acquisition system (100) according to claim 9, further comprising:

an auxiliary transmission part (903) that transmits the position information and the soil quality information stored in the storage part (506) to another working machine (1B) on the worksite; and

an auxiliary receiving part (44) that is arranged at the another working machine (1B), and receives the position information and the soil quality information transmitted from the auxiliary transmission part (43).

11. A working machine (1), comprising:

a machine body (10, 12) having a traveling part travelable on a ground;

a working attachment (20) supported by the machine body movably relative to the machine body (10, 12) for performing a predetermined work to the ground; and

the soil quality information acquisition system (100) according to any one of claims 1 to 10.

**Patentansprüche**

1. System zur Erfassung von Bodenqualitätsinformationen (100) zur Verwendung in einer Arbeitsmaschine (1), die Folgendes beinhaltet: einen Maschinenkörper (10, 12) mit einem auf einem Boden fahrbaren Fahrteil; und einen Arbeitsaufsatz (20), der an dem Maschinenkörper (10, 12) relativ zu diesem beweglich angebracht ist, um eine vorbestimmte Arbeit an dem Boden auszuführen, wobei das System zum Erfassen von Bodenqualitätsinformationen (100) angepasst ist zum Erfassen von Bodenqualitätsinformationen, bei denen es sich um Informationen über die Bodenqualität an einer Baustelle handelt, wobei das System zur Erfassung von Bodenqualitätsinformationen (100) Folgendes umfasst:

ein Positionsinformationserfassungsteil (33), das Positionsinformationen über die Arbeitsmaschine auf der Baustelle erfasst;

ein Erfassungszeitbestimmungsteil (503), das eine Erfassungszeit auf der Grundlage von bodenqualitätsrelevanten Informationen bestimmt, bei denen es sich um Informationen über eine Veränderung der Bodenqualität auf der Baustelle handelt, und das ein Erfassungssignal bezüglich der Erfassungszeit ausgibt;

ein Bodenqualitätsinformationserfassungsteil (502), das das vom Erfassungszeitbestimmungsteil (503) ausgegebene Erfassungssignal empfängt und die Bodenqualitätsinformationen als Reaktion auf das Erfassungssignal erfasst; und

ein Speicherteil (506), das die vom Positionsinformationserfassungsteil (33) erfassten Positionsinformationen und die vom Bodenqualitätsinformationserfassungsteil (502) erfassten Bodenqualitätsinformationen in Verbindung miteinander speichert.

2. System zur Erfassung von Bodenqualitätsinformationen (100) gemäß Anspruch 1, wobei das Erfassungszeitbestimmungsteil (503) einen Referenzbereich festlegt, der virtuell so geschätzt wird, dass er eine bestimmte Bodenqualität auf der Baustelle aufweist, und die Erfassungszeit auf der Grundlage einer relativen Position der Arbeitsmaschine (1) zu dem Referenzbereich bestimmt, wobei die relative Position in den bodenqualitätsrelevanten Informationen enthalten ist.

3. System zur Erfassung von Bodenqualitätsinformationen (100) gemäß Anspruch 2, wobei das Erfassungszeitbestimmungsteil (503) als den Referenzbereich einen Bereich festlegt, der durch einen Referenzkreis mit einem vorbestimmten Radius vom Mittelpunkt des Maschinenkörpers (10, 12) in einer Draufsicht zu einer vorbestimmten Referenzzeit definiert ist.

4. System zur Erfassung von Bodenqualitätsinformationen (100) gemäß Anspruch 3, wobei das Erfassungszeitbestimmungsteil (503) das Erfassungssignal ausgibt, wenn die Arbeitsmaschine (1) beim Fahren des Fahrteils einen Grenzkreis überschreitet, der innerhalb des Referenzkreises liegt und konzentrisch zum Referenzkreis ist.

5. System zur Erfassung von Bodenqualitätsinformationen (100) gemäß Anspruch 3, wobei das Erfassungszeitbestimmungsteil (503) das Erfassungssignal ausgibt, wenn sich der Arbeitsaufsatz (20) über den Referenzkreis hinaus erstreckt.

6. System zur Erfassung von Bodenqualitätsinformationen (100) gemäß Anspruch 2, wobei das Erfassungszeitbestimmungsteil (503) als Referenzbereich einen Bereich vom Maschinenkörper (10, 12) bis zu einer vorbestimmten Tiefe unter dem Boden zu einer vorbestimmten Referenzzeit festlegt.

7. System zur Erfassung von Bodenqualitätsinformationen (100) gemäß einem der Ansprüche 1 bis 6, wobei das Erfassungszeitbestimmungsteil (503) die Erfassungszeit auf der Grundlage einer verstrichenen Zeitspanne seit einer vorherigen Erfassungszeit von Bodenqualitätsinformationen durch das Bodenqualitätsinformationserfassungsteil (502) bestimmt, wobei die verstrichene Zeitspanne in den bodenqualitätsrelevanten Informationen enthalten ist.

8.  System zur Erfassung von Bodenqualitätsinformationen (100) gemäß einem der Ansprüche 1 bis 7, das ferner ein Klimainformationserfassungsteil umfasst, das konfiguriert ist, um Klimainformationen für die Baustelle zu erfassen, wobei
    der Erfassungszeitbestimmungsabschnitt (503) die Erfassungszeit auf der Grundlage der vom Klimainformationserfassungsteil erfassten Klimainformationen bestimmt, wobei die Klimainformationen in den bodenqualitätsrelevanten Informationen enthalten sind.

9.  System zur Erfassung von Bodenqualitätsinformationen (100) gemäß einem der Ansprüche 1 bis 8, wobei sich das Speicherteil (506) entfernt von der Arbeitsmaschine (1) befindet, wobei das System zur Erfassung von Bodenqualitätsinformationen (100) ferner Folgendes umfasst:

    ein Übertragungsteil (43), das die vom Positionsinformationserfassungsteil (33) erfassten Positionsinformationen und die vom Bodenqualitätsinformationserfassungsteil (502) erfassten Bodenqualitätsinformationen überträgt; und
    ein Empfangsteil (44), das sich entfernt vom Übertragungsteil (43) befindet und die vom Übertragungsteil (43) übertragenen Positionsinformationen und Bodenqualitätsinformationen empfängt und die Positionsinformationen und Bodenqualitätsinformationen in das Speicherteil (506) eingibt.

10. System zur Erfassung von Bodenqualitätsinformationen (100) gemäß Anspruch 9, das ferner Folgendes umfasst:

    ein Hilfsübertragungsteil (903), das die im Speicherteil (506) gespeicherten Positionsinformationen und Bodenqualitätsinformationen an eine andere Arbeitsmaschine (1B) auf der Baustelle überträgt; und
    ein Hilfsempfangsteil (44), das an der anderen Arbeitsmaschine (1B) angeordnet ist und die vom Hilfsübertragungsteil (43) übertragenen Positionsinformationen und Bodenqualitätsinformationen empfängt.

11. Arbeitsmaschine (1), Folgendes umfassend:

    einen Maschinenkörper (10, 12) mit einem auf dem Boden fahrbaren Fahrteil;
einen Arbeitsaufsatz (20), der von dem Maschinenkörper gehalten wird und relativ zum Maschinenkörper (10, 12) beweglich ist, um eine vorbestimmte Arbeit an dem Boden auszuführen; und
das System zur Erfassung von Bodenqualitätsinformationen (100) gemäß einem der Ansprüche 1 bis 10.

**Revendications**

1.  Système d'acquisition d'informations sur la qualité du sol (100) destiné à être utilisé dans une machine de travail (1) comportant : un corps de machine (10, 12) ayant une partie mobile pouvant se déplacer sur un sol ; et un accessoire de travail (20) fixé au corps de machine (10, 12) de manière mobile par rapport au corps de machine (10, 12) pour effectuer un travail prédéterminé sur le sol, le système d'acquisition d'informations sur la qualité du sol (100) étant adapté pour acquérir des informations sur la qualité du sol, qui sont des informations sur une qualité du sol sur un chantier, le système d'acquisition d'informations sur la qualité du sol comprenant :

    une partie d'acquisition d'informations de position (33) qui acquiert des informations de position concernant la machine de travail sur le chantier ;
    une partie de détermination du moment d'acquisition (503) qui détermine un moment d'acquisition sur la base d'informations pertinentes sur la qualité du sol, qui sont des informations liées à un changement de la qualité du sol sur le chantier, et qui émet un signal d'acquisition concernant le moment d'acquisition ;
    une partie d'acquisition d'informations sur la qualité du sol (502) qui reçoit le signal d'acquisition émis par la partie de détermination du moment d'acquisition (503) et acquiert les informations sur la qualité du sol en réponse au signal d'acquisition ; et
    une partie de stockage (506) qui stocke les informations de position acquises par la partie d'acquisition d'informations de position (33) et les informations sur la qualité du sol acquises par la partie d'acquisition d'informations sur la qualité du sol (502) en les associant les unes aux autres.

2.  Système d'acquisition d'informations sur la qualité du sol (100) selon la revendication 1, dans lequel la partie de détermination du moment d'acquisition (503) définit une zone de référence estimée virtuellement comme ayant une qualité de sol spécifique sur le chantier, et détermine le moment d'acquisition sur la base d'une position relative de la machine de travail (1) par rapport à la zone de référence, la position relative étant incluse dans les informations pertinentes sur la qualité du sol.

3. Système d'acquisition d'informations sur la qualité du sol (100) selon la revendication 2, dans lequel la partie de détermination du moment d'acquisition (503) définit, comme la zone de référence, une zone délimitée par un cercle de référence ayant un rayon prédéterminé à partir du centre du corps de la machine (10, 12) dans une vue en plan à un moment de référence prédéterminé.

4. Système d'acquisition d'informations sur la qualité du sol (100) selon la revendication 3, dans lequel la partie de détermination du moment d'acquisition (503) émet le signal d'acquisition lorsque la machine de travail (1) dépasse un cercle limite situé à l'intérieur du cercle de référence et concentrique au cercle de référence lors du déplacement de la partie mobile.

5. Système d'acquisition d'informations sur la qualité du sol (100) selon la revendication 3, dans lequel la partie de détermination du moment d'acquisition (503) émet le signal d'acquisition lorsque l'accessoire de travail (20) s'étend au-delà du cercle de référence.

6. Système d'acquisition d'informations sur la qualité du sol (100) selon la revendication 2, dans lequel la partie de détermination du temps d'acquisition (503) définit, comme la zone de référence, une plage allant du corps de la machine (10, 12) à une profondeur prédéterminée sous le sol à un moment de référence prédéterminé.

7. Système d'acquisition d'informations sur la qualité du sol (100) selon l'une quelconque des revendications 1 à 6, dans lequel la partie de détermination du temps d'acquisition (503) détermine le temps d'acquisition sur la base d'une période de temps écoulée depuis un temps d'acquisition précédent d'informations sur la qualité du sol par la partie d'acquisition d'informations sur la qualité du sol (502), la période de temps écoulée étant incluse dans les informations pertinentes sur la qualité du sol.

8. Système d'acquisition d'informations sur la qualité du sol (100) selon l'une quelconque des revendications 1 à 7, comprenant en outre une partie d'acquisition d'informations climatiques configurée pour acquérir des informations climatiques pour le chantier, dans lequel
la partie de détermination du moment d'acquisition (503) détermine le moment d'acquisition sur la base des informations climatiques acquises par la partie d'acquisition d'informations climatiques, les informations climatiques étant incluses dans les informations pertinentes sur la qualité du sol.

9. Système d'acquisition d'informations sur la qualité du sol (100) selon l'une quelconque des revendications 1 à 8, dans lequel la partie de stockage (506) est située à distance de la machine de travail (1), le système d'acquisition d'informations sur la qualité du sol (100) comprenant en outre :

une partie de transmission (43) qui transmet les informations de position acquises par la partie d'acquisition d'informations de position (33) et les informations sur la qualité du sol acquises par la partie d'acquisition d'informations sur la qualité du sol (502) ; et
une partie de réception (44) qui est située à distance de la partie de transmission (43), et qui reçoit les informations de position et les informations sur la qualité du sol transmises par la partie de transmission (43) et entre les informations de position et les informations sur la qualité du sol dans la partie de stockage (506).

10. Système d'acquisition d'informations sur la qualité du sol (100) selon la revendication 9, comprenant en outre :

une partie de transmission auxiliaire (903) qui transmet les informations de position et les informations sur la qualité du sol stockées dans la partie de stockage (506) à une autre machine de travail (1B) sur le chantier ; et
une partie de réception auxiliaire (44) qui est disposée à l'autre machine de travail (1B) et qui reçoit les informations de position et les informations sur la qualité du sol transmises par la partie de transmission auxiliaire (43).

11. Machine de travail (1), comprenant :

un corps de machine (10, 12) ayant une partie mobile pouvant se déplacer sur un sol ;
un accessoire de travail (20) supporté par le corps de machine de manière mobile par rapport au corps de machine (10, 12) pour effectuer un travail prédéterminé sur le sol ; et
le système d'acquisition d'informations sur la qualité du sol (100) selon l'une quelconque des revendications 1 à 10.

# FIG.1

# FIG.2

100

CONTROLLER 50

| 31 | MANIPULATION PART | → | DRIVE CONTROL PART 501 | → | DRIVE PART 41 |

31 MANIPULATION PART →

32 INPUT PART →

33 BODY POSITION DETECTION PART →

34 ATTACHMENT POSITION DETECTION PART →

35 CYLINDER VOLTAGE DETECTION PART →

36 PHOTOGRAPHING DEVICE →

37 CLIMATE INFORMATION ACQUISITION PART →

DRIVE CONTROL PART 501

SOIL QUALITY INFORMATION ACQUISITION PART 502

ACQUISITION TIME DETERMINATION PART 503

DETERMINATION PART 504

INPUT INSTRUCTION PART 505

STORAGE PART 506

→ DRIVE PART 41

→ DISPLAY PART 42

→ TRANSMISSION PART 43

SERVER 90

SERVER RECEIVING PART 901

SERVER STORAGE PART 902

EP 4 310 256 B1

# FIG.3

FRONT

LEFT ⟷ RIGHT

REAR

P

1

# FIG.4

EP 4 310 256 B1

# FIG.5

```
        START

          │
          ▼                    S1
┌──────────────────────┐
│  ACQUIRE POSITION    │
│ INFORMATION ABOUT    │
│ MACHINE MAIN BODY    │
└──────────────────────┘
          │
          ▼                    S2
┌──────────────────────┐
│ ACQUIRE SOIL QUALITY │
│    INFORMATION       │
└──────────────────────┘
          │
          ▼                    S3
┌──────────────────────┐
│  STORE POSITION      │
│ INFORMATION AND SOIL │
│ QUALITY INFORMATION  │
└──────────────────────┘
          │
          ▼

         END
```

# FIG.6

START

EXCAVATOR HAS MOVED AT DISTANCE Q OR LONGER ? — S11

NO

YES

ACQUIRE SOIL QUALITY INFORMATION — S12

STORE POSITION INFORMATION AND SOIL QUALITY INFORMATION — S13

END

FIG.7

```
                    ┌─────────────┐
                    │    START    │
                    └─────────────┘
                           │
                           ▼
                      ╱─────────╲      S21
                   ╱   EXCAVATOR   ╲  NO
                ╱  HAS MOVED AT DISTANCE ╲──────────────────┐
                ╲     Q OR LONGER     ╱                     │
                   ╲       ?      ╱                         │
                      ╲─────────╱                           │
                       YES│◄──────────────┐                 ▼
                          ▼            S22 │            ╱─────────╲    S24
                 ┌──────────────────┐  YES │         ╱  TIME PERIOD  ╲
                 │ACQUIRE SOIL QUALITY│     └────────╲  T OR LONGER   ╱
                 │   INFORMATION    │              ╲   ELAPSES?  ╱
                 └──────────────────┘                 ╲─────────╱
                          │        S23                     │NO
                          ▼                                │
                 ┌──────────────────┐                      │
                 │ STORE POSITION   │                      │
                 │INFORMATION AND SOIL│                     │
                 │QUALITY INFORMATION│                      │
                 └──────────────────┘                      │
                          │◄──────────────────────────────┘
                          ▼
                    ┌─────────────┐
                    │     END     │
                    └─────────────┘
```

FIG.8

```
                    ┌─────────────┐
                    │    START    │
                    └─────────────┘
                           │
                           ▼
                          S31
              ╱─────────────────────╲
             ╱      EXCAVATOR         ╲        NO
            ⟨  HAS MOVED AT DISTANCE   ⟩─────────────────┐
             ╲      Q OR LONGER       ╱                   │
              ╲          ?           ╱                    │
               ╲───────────────────╱                     │
                      YES│◄──────────────┐                │
                         │               │                ▼
                         ▼              S32              S34
              ┌────────────────────┐     │       ╱─────────────────────╲
              │ ACQUIRE SOIL QUALITY│    │      ╱                       ╲
              │    INFORMATION      │    │ YES ⟨   CLIMATE CHANGES?      ⟩
              └────────────────────┘     └─────╲                       ╱
                         │                       ╲─────────────────────╱
                         ▼              S33             NO│
              ┌────────────────────┐                      │
              │  STORE POSITION     │                      │
              │ INFORMATION AND SOIL│                      │
              │ QUALITY INFORMATION │                      │
              └────────────────────┘                      │
                         │◄─────────────────────────────────┘
                         ▼
                    ┌─────────────┐
                    │     END     │
                    └─────────────┘
```

22

# FIG.9

FIG.10

EP 4 310 256 B1

# FIG.11

RIGHT

FRONT ◄──►REAR

LEFT

23    22B

22A

θ

21

12

EP 4 310 256 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019163621 A
- JP 2018111950 A